Europäisches Patentamt

European Patent Office

Office européen des brevets

(11) Veröffentlichungsnummer: **0 257 222**
**A1**

(12) # EUROPÄISCHE PATENTANMELDUNG

(21) Anmeldenummer: **87108786.2**

(22) Anmeldetag: **19.06.87**

(51) Int. Cl.⁴: **A61F 2/30** , A61F 2/36

(30) Priorität: **09.08.86 DE 3627097**

(43) Veröffentlichungstag der Anmeldung:
**02.03.88 Patentblatt 88/09**

(84) Benannte Vertragsstaaten:
**AT BE CH DE ES FR GB GR IT LI LU NL SE**

(71) Anmelder: **S + G IMPLANTS GMBH**
**Grapengiesserstrasse 34**
**D-2400 Lübeck(DE)**

(72) Erfinder: **Grundei, Hans,**
**Hamburger Strasse 89**
**D-2400 Lübeck(DE)**
Erfinder: **Henssge, Joachim,**
**Im Trentsaal 7**
**D-2400 Lübeck(DE)**

(74) Vertreter: **Wilcken, Thomas, Dipl.-Ing. et al**
**Musterbahn 1**
**D-2400 Lübeck(DE)**

(54) **Stiel für Gelenk-Endoprothesen.**

(57) Der Stiel (1) für Gelenk-Endoprothesen ist mindestens über einen Teil seiner Länge hohl ausgebildet, wobei der Hohlraum (2) durch wenigstens einen Längsschlitz (3) mit der Umgebung verbunden ist.

Fig.2

Fig.3

## Stiel für Gelenk-Endoprothesen

Die Erfindung bezieht sich auf einen in den Markraum eines Knochens einsetzbaren Stiel für Gelenk-Endoprothesen.

Bekannte Stiele für Gelenk-Endoprothesen aus Metall oder einem widerstandsfähigen Kunststoff sind im allgemeinen vollwandig ausgebildet oder sind in Längsrichtung zusätzlich mit Rippen oder Nuten versehen, durch die ihre Steifigkeit vergrößert wird. Daher besitzen solche Stiele eine von dem natürlichen Knochen abweichende Elastizität. Dadurch, daß die bekannten Stiele zu starr ausgebildet sind und der natürliche Knochen eine höhere Elastizität besitzt, treten durch die ständigen Wechselbelastungen an Gelenkprothesen Ermüdungserscheinungen auf, die zu Brüchen führen können.

Die Aufgabe der Erfindung besteht darin, Stiele für Gelenk-Endoprothesen so auszubilden, daß ihre Elastizität der des natürlichen Knochens angepaßt bzw. angenähert ist.

Diese Aufgabe wird nach der Erfindung bei den eingangs erwähnten Stielen dadurch gelöst, daß der Stiel mindestens über einen Teil seiner Länge hohl ausgebildet ist und wenigstens einen Längsschlitz aufweist, der in den Hohlraum mündet und eine Verbindung des Hohlraumes mit der Umgebung bildet. Der Hohlraum des Stieles ist weiter an einem Ende in Achsrichtung, das heißt am freien Ende offen.

Vorteilhaft besteht der Stiel aus einem körperverträglichen Metall und ist mit einer Außenschicht aus offenzelligem bzw. porösem Metall versehen, wodurch der Knochen beim Implantieren des Schaftes in dem Markraum gereizt wird, was zu einer Bildung von Knochengewebe führt, die sich bis in den Hohlraum des Schaftes erstreckt und anschließend zur Bildung spongiöser Knochenzellen führt.

Durch die Ausbildung des Stieles für Gelenk-Endoprothesen wird seine Elastizität wesentlich vergrößert und wird der Elastizität des natürlichen Knochens angepaßt oder wenigstens angenähert, so daß infolge der ständigen Wechselbelastungen Brüche nicht oder kaum noch auftreten können. Weiter wird das Gewicht der Prothese wesentlich herabgesetzt und der Verbrauch an teurem Material, insbesondere Metall verringert.

Die Erfindung mit weiteren vorteilhaften Merkmalen wird nachstehend anhand der Zeichnung beschrieben. Es zeigen:

Figur 1 eine perspektivische Ansicht des Stieles für eine Hüftgelenk-Endoprothese,

Figur 2 eine mediale Ansicht des Stieles nach Fig. 1 gesehen in Richtung des Pfeiles x,

Figur 3 einen Querschnitt nach Linie III bis III der Fig. 2,

Figur 4 eine Ansicht des Stieles in Richtung des Pfeiles Y der Fig. 2,

Figur 5 eine Ansicht eines Tibia-Stieles einer Kniegelenk-Endoprothese,

Figur 6 eine Seitenansicht zu Fig. 5,

Figur 7 einen Querschnitt nach Linie VII bis VII der Fig. 5,

Figur 8 eine Endansicht zu Fig. 6, gesehen in Richtung des Pfeiles y.

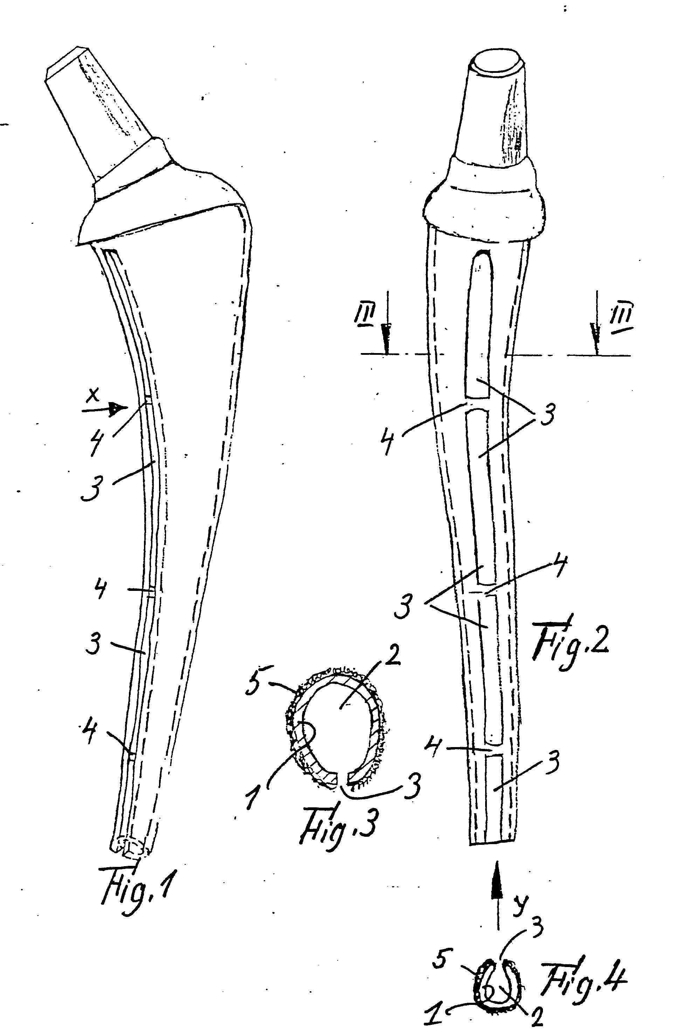

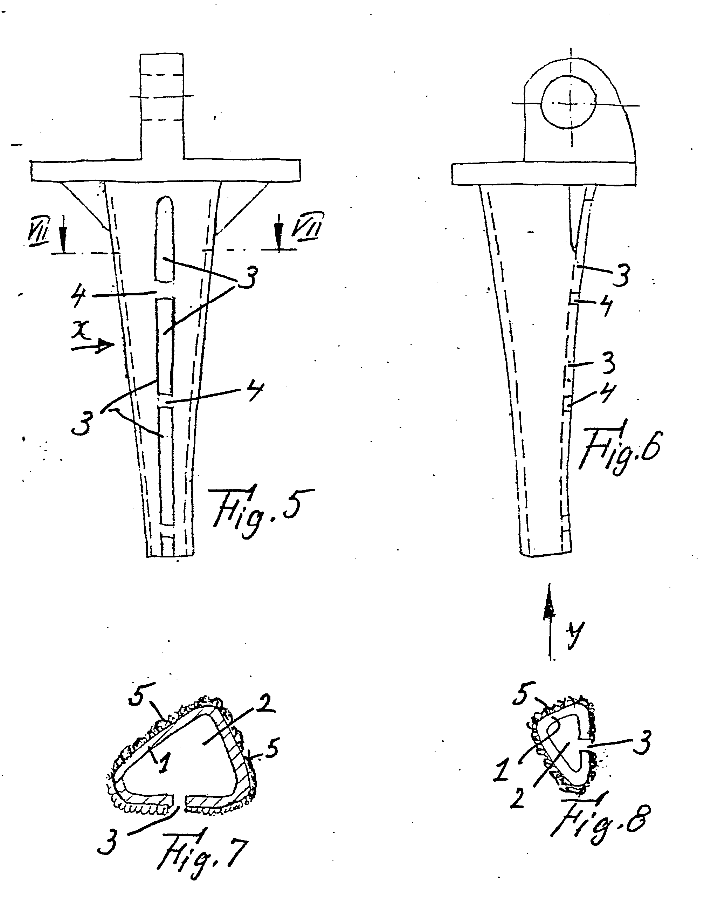

Der Stiel 1 für eine Hüftgelenk-Endoprothese nach den Fig. 1 bis 4 ist mit einem über seine Länge verlaufenden, unten offenen (Fig.4) Hohlraum 2 versehen, der durch einen Schlitz 3 mit der Umgebung verbunden ist. Der Schlitz 3 kann zur Anpassung an die Elastizität des Markknochens an einer oder mehreren Stellen mit einem Quersteg 4 überbrückt sein, der die beiden Schenkel des Stielquerschnittes verbindet.

Vorteilhaft besteht der Stiel 1 aus einem körperverträglichem Metall, mit dem eine Außenschicht 5 aus offenzelligem bzw. porösem Metall verschweißt ist. Diese Außenschicht 5 führt zum Einwachsen von Knochengewebe und weiter dazu, daß dieses Gewebe von der Außenschicht ausgehend über den Schlitz 3 auch in den Hohlraum 2 einwächst und zu spongiöser Knochenbildung führt.

Der den Hohlraum 2 mit der Umgebung verbindende Schlitz 3 wird vorteilhaft auf der medialen Seite des Stieles 1 vorgesehen, um dadurch die Elastizität des Stieles zu vergrößern. Es können aber auch mehrere längsverlaufende Schlitze 3 vorgesehen sein.

Ebenso wie der Stiel nach den Fig. 1 bis 4 für eine Hüftgelenk-Endoprothese können auch die Stiele für den Tibia teil (Fig. 5 bis 8) und auch die Stiele für den Femurteil einer Kniegelenk-Endoprothese ausgebildet sein, wobei die Bezugszeichen nach den Fig. 1 bis 4 auch für entsprechende Teile der Fig. 5 bis 8 verwendet worden sind, so daß zu den Fig. 5 bis 8 keine weiteren Erläuterungen erforderlich sein dürften.

## Ansprüche

1. In den Markraum eines Knochens einsetzbarer Stiel für Gelenkendoprothesen, dadurch gekennzeichnet, daß der Stiel mindestens über einen Teil seiner Länge hohl ausgebildet ist und wenigstens einen Längsschlitz (3) aufweist, der in den Hohlraum (2) mündet und eine Verbindung des Hohlraumes mit der Umgebung bildet.

2. Stiel nach Anspruch 1, dadurch gekennzeichnet, daß der vollmetallische, mit dem Hohlraum (2) versehene Stiel (1) mit einer Außenschicht (5) aus offenzelligem bzw. porösem Metall verschweißt ist.

3. Stiel nach Anspruch 1 oder 2, dadurch gekennzeichnet, daß der Hohlraum (2) an einem Ende des Stieles in Achsrichtung offen ist.

4. Stiel nach einem der Ansprüche 1 bis 3, dadurch gekennzeichnet, daß der Schlitz (3) an der medialen Seite des Stieles vorgesehen ist.

5. Stiel nach einem der Ansprüche 1 bis 4, dadurch gekennzeichnet, daß der Schlitz (3) durch wenigstens einen Quersteg (4) überbrückt ist, der die beiden Schenkel des Stielquerschnittes verbindet.

x

4

3

4

3

4

Fig. 1

5

2

1

3

Fig. 3

III        III

4

3

4

3

3

4

Fig. 2

4

3

y

3

5

1        2

Fig. 4

0 257 222

Fig.5

Fig.6

Fig.7

Fig.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 438 469 (ETABLISSEMENTS TORNIER) * Figuren 1-3; Seite 1, Zeilen 32-39; Seite 2, Zeilen 1-12,28-33 * | 1,3,4 | A 61 F 2/30 A 61 F 2/36 |
| Y | | 2 | |
| | --- | | |
| X | FR-A-2 104 009 (R.F. HOCHMAN) * Figuren 2-4; Seite 3, Zeile 29 - Seite 4, Zeile 15 * | 1,3-5 | |
| | --- | | |
| Y | EP-A-0 075 378 (CRUCIBLE INC.) * Zusammenfassung * | 2 | |
| | ----- | | |

RECHERCHIERTE
SACHGEBIETE (Int. Cl.4)

A 61 F

Der vorliegende Recherchenbericht wurde für alle Patentansprüche erstellt.

| Recherchenort | Abschlußdatum der Recherche | Prüfer |
|---|---|---|
| DEN HAAG | 17-11-1987 | ARGENTINI A. |